Europäisches Patentamt

European Patent Office

Office européen des brevets

(19)

(11) Veröffentlichungsnummer: **0 167 670**
**B1**

(12) **EUROPÄISCHE PATENTSCHRIFT**

(45) Veröffentlichungstag der Patentschrifft:
28.02.90

(21) Anmeldenummer: 84115312.5

(22) Anmeldetag: 12.12.84

(51) Int. Cl.⁵: **A 61 N  5/02, A 61 B  17/22**

(54) Einrichtung zum Zertrümmern von im Körper eines Lebewesens befindlichen Konkrementen.

(30) Priorität: 13.07.84 DE 3425897

(43) Veröffentlichungstag der Anmeldung:
15.01.86 Patentblatt 86/03

(45) Bekanntmachung des Hinweises auf die Patenterteilung:
28.02.90 Patentblatt 90/09

(84) Benannte Vertragsstaaten:
DE NL SE

(56) Entgegenhaltungen:
EP-A-0 081 639
DE-A-2 508 494
DE-A-2 628 234
FR-A-2 269 356
FR-A-2 289 211
FR-A-2 409 053
US-A-4 397 313
US-A-4 682 600

IEEE PROCEEDINGS ON MICROWAVE THEORY AND TECHNIQUES, Band MTT-19, Nr. 2, Februar 1971, Seiten 146-152, New York, US; H.P. SCHWAN: "Interaction of microwave and radio frequency radiation with biological systems"

(73) Patentinhaber: Siemens Aktiengesellschaft
Wittelsbacherplatz 2
D-8000 München 2 (DE)

(72) Erfinder: Haas, Werner, Dr.
Ringstrasse 34
D-8525 Uttenreuth (DE)
Erfinder: Knüpfer, Wolfgang, Dr.
Rudelsweiherstrasse 11
D-8520 Erlangen (DE)
Erfinder: Pfeiler, Manfred, Dr.
Ludwig-Thoma-Strasse 25
D-8520 Erlangen (DE)

EP 0 167 670 B1

**Beschreibung**

Die Erfindung betrifft eine Einrichtung gemäß dem ersten Teil des Patentanspruches 1.

Es ist bekannt, Konkremente, z. B. Nierensteine, durch Ultraschallschockwellen zu zertrümmern (DE-A-3 122 056). Dabei wird ein Grenzflächeneffekt zwischen Körpergewebe und Steinstruktur ausgenutzt. Entsprechend der Reflexion der eintreffenden Stoßwelle an der Vorder- oder Hintergrenzfläche des Konkrementes werden auf dieses Druck- bzw. Zugkräfte ausgeübt, die zur Zertrümmerung führen. In der Praxis erfolgt dabei die Ankopplung des menschlichen Körpers an den Stoßwellengenerator durch ein Wasserbad, das die erzeugten Stoßwellen überträgt. Dieses Wasserbad dient auch zur Auskopplung der Ultraschallenergie, die den Steinbezirk passiert hat.

Das Wasserbad stellt ein Erschwernis für die Anwendung und zwar sowohl hinsichtlich der Patientenlagerung als auch hinsichtlich des Einsatzes eines Ortungssystems (Röntgensystem) dar.

Der Erfindung liegt die Aufgabe zugrunde, eine Einrichtung der eingangs genannten Art so auszubilden, daß auf das Wasserbad als Ankopplungsmittel verzichtet werden kann.

Diese Aufgabe ist erfindungsgemäß gelöst durch die Merkmale des zweiten Teiles des Patentanspruches 1. Durch die Hochfrequenzimpulse wird die Polarisierung im zu zertrümmernden Konkrement ständig geändert. Die Größer des Kraftvektors nimmt dabei in dem Bereich, in dem die lineare Ausdehnung des Konkrementes kleiner als die reziproke Frequenz des Wechselfeldes ist, mit zunehmender Frequenz zu. Die demgemäß auf das Konkrement ausgeübten elektrischen Kräfte führen über Zugbeanspruchung zu einer Zertrümmerung. Da im elektromagnetischen Wechselfeld das zu zertrümmernde Konkrement eine endliche Leitfähigkeit besitzt und in diesem Ströme induziert werden, treten magnetische Kräfte auf, die ebenso zur Zertrümmerung beitragen können. Bei der Zertrümmerung von Nierensteinen haben z. B. Zugkräfte den Vorteil, daß sie um etwa eine Größenordnung schwächer sein können als Druckkräfte, um denselben Zertrümmerungeffekt zu bewirken. Die Körpersubstanz des Patienten stellt dabei mit Ausnahme des zu zertrümmernden Konkrementes ein weitaus homogeneres Gebilde dar als für eine Ultraschallstoßwelle. Eine unbeabsichtigte Verletzung von Körperstrukturen mit ausgeprägten Grenzflächen, z. B. von Knochen, ist daher wesentlich weniger wahrscheinlich als bei der bekannten Einrichtung mit Ultraschallstoßwelle. Durch die Fokussierung einer über einen wesentlichen Teil der Körperperipherie erzeugten elektromagnetischen Welle auf das Konkrement treten nicht nur Kräfte an dessen Grenzflächen auf, sondern es wird auch in dessen Innerem Wärme erzeugt, die die Zertrümmerung unterstützt.

Durch die DE-A-2 508 494 ist es bereits bekannt, Gewebe mit elektromagnetischen Wellen zu beaufschlagen. Das Gewebe soll dabei aber nicht zerstört, sondern erwärmt werden. Die bekannte Einrichtung ist deshalb zur Erzeugung von Stoßwellen im Inneren von Konkrementen nicht geeignet.

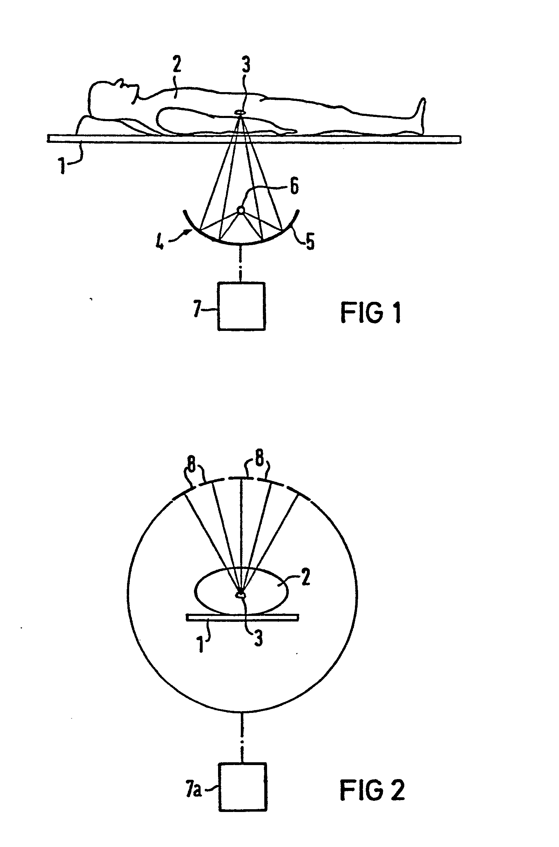

Die Erfindung ist nachfolgend anhand zweier in den Figuren 1 und 2 dargestellter Ausführungsbeispiele näher erläutert.

In der Figur 1 ist eine Patientenliege 1 dargestellt, auf der ein Patient 2 liegt, der ein Konkrement 3, z. B. einen Nierenstein, hat. Zur Zertrümmerung des Konkrementes 3 ist eine Sendeantenne 4 vorgesehen, die im Brennpunkt eines Hohlspiegels 5 einen Sender 6 für den Aufbau eines elektromagnetischen Feldes aufweist. Die vom Sender 6 ausgesandten elektromagnetischen Wellen werden durch entsprechende Lagerung des Patienten 2 relativ zur Sendeantenne 4 auf das Konkrement 3 fokussiert. Der Sender 6 wird von einem Hochfrequenzgenerator 7 gespeist.

Bei der Ausführungsform gemäß Figur 2, bei der die Teile 1, 2, 3 im Querschnitt dargestellt sind, wird der Patient 2 von einem Array aus einzelnen Antennenelementen 8 mit geeigneter dielektrischer Anpassung an das Körpermedium umgeben, die auf einen Fokus, und zwar durch entsprechende Lagerung des Patienten 2 auf das Konkrement 3 ausgerichtet sind. Die Speisung der Antennenelemente 8 erfolgt durch einen Hochfrequenzgenerator 7a.

Die Sendeantennen 4, 8 und die zugeordneten Hochfrequenzgeneratoren 7, 7a können für die Aussendung von Wellen mit breitem Frequenzspektrum ausgebildet sein. Die Stoßwellen können von Kochfrequenzimpulsen mit spektraler Betonung in einem optimalen Bereich gebildet sein. Dieser optimale Frequenzbereich ergibt sich aus der Berücksichtigung der Zunahme der Kräftewirkung und der Abnahme der Eindringtiefe mit der Frequenz.

Auch bei dem Ausführungsbeispiel gemäß Figur 2 können die Antennenelemente 8 Hornantennen, Rechteckhohlleiter oder dem Stand der Technik entsprechende Antennen sein. Es eignet sich als Sender z. B. ein Mikrowellenoszillator je nach erforderlichem Frequenzbereich.

**Patentansprüche**

1. Einrichtung mit einer Sendeantenne (4, 8) zum Aussenden von auf einen Bereich (3) im Körper eines Lebewesens (2) fokussierten elektromagnetischen Hochfrequenzimpulsen, die über ein geeignetes dielektrisches Material an das Körpermedium angepaßt ist, *dadurch gekennzeichnet*, daß die Einrichtung so ausgebildet ist, daß die von ihr erzeugten Hochfrequenzimpulse im Inneren von Konkrementen Stoßwellen erzeugen, die zur Zertrümmerung dieser Konkremente führen.

2. Einrichtung nach Anspruch 1, *dadurch gekennzeichnet*, daß die Antenne (4) einen Reflektor (5) und einen in dessen Brennpunkt angeordneten Sender (6) für elektromagnetische Wellen aufweist.

3. Einrichtung nach Anspruch 1, *dadurch gekennzeichnet*, daß die Sendeantenne als das zu behandelnde Objekt (2) umschließendes Array aus einzelnen Antennenelemente (8) aufgebaut ist, welche auf den Fokus ausgerichtet sind.

4. Einrichtung nach einem der Ansprüche 1 bis 3, *dadurch gekennzeichnet*, daß die Sendeantenne (4, 8) für die Aussendung von Hochfrequenzimpulsen mit breitem Frequenzspektrum ausgebildet ist.

5. Einrichtung nach einem der Ansprüche 1 bis 3, *dadurch gekennzeichnet*, daß die Sendeantenne (4, 8) für die Aussendung von Hochfrequenzimpulsen mit spektraler Betonung in einem bestimmten Bereich ausgebildet ist.

**Claims**

1. Device having a transmission antenna (4, 8) for transmitting electromagnetic high frequency impulses focussed on a region (3) in the body of a living being (2), this antenna being matched to the body medium by means of a suitable dielectric material, characterised in that the device is constructed so that the high frequency impulses produced by it generate shock waves inside calculi which lead to the disintegration of these calculi.

2. Device according to claim 1, characterised in that the antenna (4) has a reflector (5) with a transmitter (6) for electromagnetic waves arranged at its focus.

3. Device according to claim 1, characterised in that the transmission antenna is constructed from individual antenna elements (8) as an array enclosing the object (2) to be treated, the antenna elements (8) being directed to the focus.

4. Device according to one of claims 1 to 3, characterised in that transmission antenna (4, 8) is constructed for the transmission of high frequency impulses with a wide frequency spectrum.

5. Device according to one of claims 1 to 3, characterised in that transmission antenna (4, 8) is constructed for transmitting high frequency impulses with spectral concentration in a specific region.

**Revendications**

1. Dispositif comprenant une antenne émettrice (4, 8) pour émettre des impulsions électromagnétiques à haute fréquence concentrées sur une région (3) situées dans le corps d'un être vivant (2), dispositif qui est adapté par un matériau diélectrique approprié au milieu corporel, caractérisé en ce qu'il est réalisé de manière que les impulsions à haute fréquence produites par lui génèrent des ondes au sein de concrétions, qui conduisent à la fragmentation de ces concrétions.

2. Dispositif selon la revendication 1, caractérisé en ce que l'antenne (4) possède un réflecteur (5) et un émetteur (6) disposé au foyer du réflecteur pour l'émission d'ondes électromagnétiques.

3. Dispositif selon la revendication 1, caractérisé en ce que l'antenne émettrice est réalisée comme un système d'éléments d'antenne (8) séparés qui sont orientés sur le foyer, le système entourant l'objet (2) à traiter.

4. Dispositif selon une des revendications 1 à 3, caractérisé en ce que l'antenne émettrice (4, 8) est réalisée pour l'émission d'impulsions à haute fréquence dans un large spectre de fréquences.

5. Dispositif selon une des revendications 1 à 3, caractérisé en ce que l'antenne émettrice (4, 8) est réalisée pour l'émission d'impulsions à haute fréquence avec accentuation spectrale dans un domaine déterminé.

FIG 1

FIG 2